Europäisches Patentamt

European Patent Office

Office européen des brevets

(11) Publication number: **0 328 230**
**A2**

(12) # EUROPEAN PATENT APPLICATION

(21) Application number: **89200670.1**

(22) Date of filing: **19.11.81**

(51) Int. Cl.4: **C11C 3/08 , C11C 3/10 ,**
**C11C 3/12**

(43) Date of publication of application:
**16.08.89 Bulletin 89/33**

(60) Publication number of the earlier application in
accordance with Art.76 EPC: **0 079 986**

(84) Designated Contracting States:
**CH DE FR GB LI NL**

(71) Applicant: **FUJI OIL COMPANY, LIMITED**
**No. 6-1, Hachiman-cho Minami-ku**
**Osaka-shi Osaka(JP)**

(72) Inventor: **Matsuo, Takaharu**
**973-34, Shindachi Okanaka**
**Sennan-shi Osaka-fu(JP)**
Inventor: **Sawamura, Norio**
**28-10 Fomob Kumatori-cho**
**Sennan-gun Osaka-fu(JP)**
Inventor: **Hashimoto, Yukio**
**No 808-399 Higashigaoka**
**Kishiwada-shi Osaka-fu(JP)**
Inventor: **Hashida, Wataru**
**No. 7-16, Ikue 3-chome Asahi-ku**
**Osaka-shi Osaka-fu(JP)**

(74) Representative: **Allard, Susan Joyce et al**
**BOULT, WADE & TENNANT 27 Furnival Street**
**London EC4A 1PQ(GB)**

(54) **Method for the modification of fats and oils.**

(57) A method for the modification of a fat or oil wherein a mixture (mixture A) containing a glyceride-type fat or oil to be modified (material A) and a fatty acid or a nonglyceride-type ester thereof (material B) is selectively transesterified in the presence of a catalyst having a selective transesterification activity (catalyst A); the resulting fat or oil (resultant A), fatty acid or nonglyceride-type ester thereof (resultant B) and catalyst (catalyst B) are recovered from the reaction mixture; a hard butter is produced from the resultant A, and a part or all of the resultant B is hydrogenated and the hydrogenated product (material C) is reused as a part of the mixture A.

EP 0 328 230 A2

# METHOD FOR THE MODIFICATION OF FATS AND OILS

The present invention relates to a method for the modification of fats and oils. More particularly, the present invention relates to a method in which fats and oils are modified by transesterification to give fats and oils which are suitable for a hard butter such as a cacao butter substitute.

Transesterification is one of the fundamental techniques for modifying fats and oils and it has been known that transesterification can be carried out by using a metallic catalyst, such as an alkaline metal catalyst or the like.

It has recently been proposed that transesterification can be carried out by using a lipolytic enzyme. According to this enzymatic transesterification, a selective transesterification reaction can be effected and a fatty acid residue at a specific position in a glyceride can be readily interchanged with another appropriate fatty acid residue. The enzymatic transesterification makes it possible to modify fats and oils for various purposes. Furthermore, although the hydrolysis of fats and oils also occurs in the enzymatic transesterification, techniques for reducing the formation of hydrolyzates, particularly, diglycerides to a large extent such as not more than several percent have been developed (for example, US Patent No.4,268,527). Hence,the practical value of enzymatic transesterification in the production of a hard butter has increased.

In general, the enzymatic transesterification is carried out by reacting a fat or oil to be modified with an appropriate fatty acid source, such as a fatty acid or a nonglyceride-type ester thereof, in the presence of a lipolytic enzyme to selectively interchange a fatty acid residue at a specific position of the fat or oil with that of the fatty acid source, removing the enzyme and then recovering the resulting transesterified fat or oil from the reaction mixture.

However, a selective transesterification reaction is to randomize or unify the distribution of fatty acid residues among reaction sites of reactants. Therefore, when the difference between a fatty acid component at a reaction site of a fat or oil to be modified and that of a desired fat or oil is greater, a larger amount of a fatty acid source is needed. Furthermore, it is difficult to reuse a fatty acid or an ester thereof present in a reaction mixture as a fatty acid source after separation of the desired fat or oil, since the fatty acid composition thereof is quite different from that of the fatty acid source initially used and hence, a large amount of the fatty acid source is also needed.

Moreover, as mentioned above, hydrolysis of a fat or oil cccurs in the enzymatic tranesterification and it lowers the yield and reduces the quality of the desired glyceride due to the miscibility thereof with hydrolyzates. Although this problem can be solved by carrying out the selective transesterification reacation in a dry reaction system, it still requires a reduced reaction time.

We have now found that when a fatty acid or an ester thereof in a reaction mixture is recovered after a selective transesterification reaction, hydrogenated and then used again as all or a part of a fatty acid sourse, it is possible to decrease the amount of the fatty acid source to be used, to improve the yield and quality of the desired hard butter product and to reduce the reaction time.

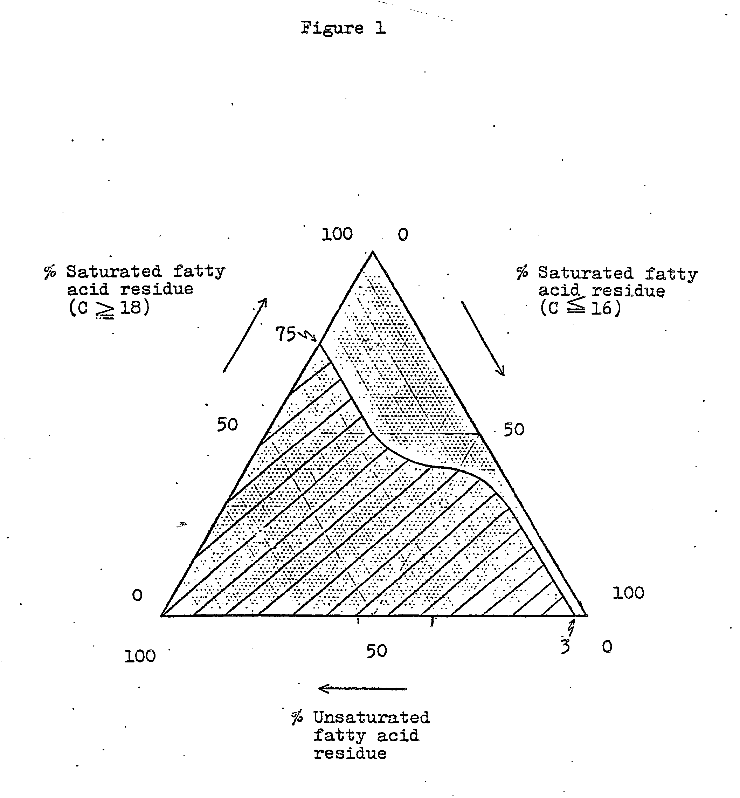

Accordingly, the present invention provides a method for the modification of a fat or oil wherein a mixture (mixture A) containing a glyceride-type fat or oil to be modified (material A) and a fatty acid or a nonglyceride-type ester thereof (material B) is selectively transesterified in the preence of a catalyst having a selective transesterification activity (catalyst A); the resulting fat or oil (resultant A), fatty acid or nonglyceride-type ester thereof (resultant B) and catalyst (catalyst B) are recovered from the reaction mixture; and a hard butter is produced from the resultant A, and a part or all of the resultant B is hydrogenated and the hydrogenated product (material C) is reused as a part of the mixture A. The present invention will be further described with reference to the accompanying drawing wherein Figure 1 is a triangle showing a fatty acid composition at the $\alpha$- positions (1 and 3-positions) of a fat or oil to be modified according to the method of the present invention. In the method of the present invention, all or a low melting point fraction of the resultant A may be also reused as a part of the mixture A, and /or a fatty acid having not more than 16 carbon atoms or an ester thereof may be removed from the resultant B or the material C.

The material A used in the method of the present invention is a glyceride-type fat or oil and, preferably, is rich in oleic acid residues at the $\beta$-position (2-position) thereof. More particularly, the fatty acid residues at the $\beta$ -position of the material A are preferably composed of not less than 70 % of oleic acid residue. When the amount of oleic acid residues at the $\beta$-position is too small, it is difficult to obtain a hard butter suitable for use as a cacao butter substitute, which is rich in oleic acid residues at the $\beta$-position, in good yield even if a selective transesterification at the $\alpha$-position is effected. It is preferable that the fatty acid residues at the $\alpha$-position of the material A consist essentially of those derived from a fatty acid having from 14 to 22 carbon atoms. More particularly, the fatty acid residue composition at the $\alpha$-position is preferably

within the shaded part of Figure 1. In Figure 1, the points of the triangle show that the fatty acid residue composition is composed of 100 % of the residues derived from a saturated fatty acid having not less than 18 carbon atoms (e.g. stearic acid, arachic acid, behenic acid, in general, mainly stearic acid), 100 % of residues derived from a saturated fatty acid having not more than 16 carbon atoms (e.g. palmitic acid, myristic acid, in general, mainly palmitic acid), or 100 % of residues derived from an unsaturated fatty acid (in general, mainly an unsaturated fatty acid having 18 carbon atoms such as oleic acid, linolic acid, linolenic acid, etc.), respectively. Within the shaded part, since the amount of the residues derived from an unsaturated fatty acid is greater, more desirable effects can be obtained by hydrogenating the resultant B and reusing it as a part of the mixture A. Examples of the material A are olive oil, oleic safflower oil, camellia oil, palm oil, rapeseed oil (Zero Erucic type), shea butter, sal fat, mango fat, kokum butter, Borneo tallow, Malabar tallow and a fractionated fat thereof. However, when the fatty acid residue composition of the fat is outside the shaded part of Figure 1, for example, when the fat to be modified contains more than 80 % of 2-oleo-1,3-distearin (hereinafter referred to as SOS) or more than 98 % of 2-oleo-1,3-dipalmitin (hereinafter referred to as POP), the effect of the method of the present invention is decreased.

The material B is a fatty acid source and, preferably, contains residues of palmitic acid or stearic acid. In general, the fatty acid residues in material B are composed of not less than 50 % of palmitic and stearic acid residues, the palmitic acid residues being at the most 1.5 times as much as the stearic acid residue by weight. The material B may be in the form of a free fatty acid or an ester thereof. The ester should be separable from a triglyceride, that is, it should be a nonglyceride-type ester since, if the ester is, for example, a diglyceride, which forms an eutectic mixture with a triglyceride and cannot be separated from the triglyceride by conventional industrial operations, it remains in a hard butter product which causes a deterioration of the quality of the product. Examples of nonglyceride-type ester are monohydric alcohol esters, dihydric alcohol esters such as propylene glycol esters and sorbitan esters. In the present invention, since the content of the resultant B in the reaction mixture is higher than that of fatty acids in a conventional fat or oil to be subjected to refining, separation of the resultant A from the resultant B is readily effected when the resultant B is a monohydric alcohol ester rather than a free fatty acid. Therefore, the material B is preferably a fatty acid monohydric alcohol ester, particularly, one in which the alcohol moiety thereof has 1 to 4 carbon atoms. Furthermore, when the method of the present invention includes a step for fractionating different fatty acid esters as mentioned hereinafter, it is preferable to use a fatty acid monohydric alcohol ester as the material B since the fractionation can be readily carried out.

The mixture A containing the materials A and B may further contain a solvent such as n-hexane so as to make it liquid. When the water content of the mixture A is high, hydrolysis occurs at the same time as the tranesterification reaction. The formation of a diglyceride which is difficult to separate from the desired triglyceride is increased and thus, the resultant A cannot be used in the production of a hard butter in which a solid fat index (SFI) curve, a cooling curve of the resultant A and a tempering operation are of importance. It is therefore preferable to carry out the tranesterification reaction in as dry a state as possible and the water content of the transesterification reaction system, including that of the solvent to be used and the catalyst, is preferably not more than o.18% by weight based on the total weight of the materials A and B.

The catalyst A to be used in the present invention shows a superior activity in a dry reaction system without the addition of water as mentioned above. A typical example of the catalyst A is that prepared by dispersing, absorbing or bonding a lipolytic enzyme (lipase) in or on a carrier in an aqueous system and drying the resulting mixture at an adequately slow initial drying rate (European Patent Application No.35883). Although the activity is somewhat weak, a cell-bond lipolytic enzyme (exocellular lipolytic enzyme) can also be used without any specific treatment. The catalyst A for use in the present invention may be any suitable catalyst providing that it shows the transesterification activity and the desired selectivity in the dry reaction system. The selectivity of the transesterification reaction is directed to the $\alpha$-position of the triglyceride which means that no substantial transesterification occurs at the $\beta$- position. Examples of suitable catalysts A having such selectivity are lipases derived from animals or vegetables such as pancreas lipase and rice bran lipase; and lipases derived from microbes such as those originated from Rhizopus delemar, Rhizopus japonicus, Rhizopus niveus, Asperigillus nigar, Mucor javanicus.

The transesterification reaction may be carried out at a temperature in the range of from 20 to 75° C according to a standard method.

In order to separate the resultants A and B from the reaction mixture, a conventional technique such as distillation, adsorption or fractionation can be employed. Usually, distillation is convenient. The resultant A can be used in the production of a hard butter as such or may be subjected to fractionation to remove a high melting point fraction and/or a low melting point fraction and then used in the production of a hard butter. This fractionation is well known in the art. In general, when a hard butter is produced from the resultant A alone, the quality of the hard butter is inversely proportional to yield thereof.

According to the present invention, the resultant B is hydrogenated and the hydrogenated product (material C) is reused as a part of the mixture A. This makes it possible to increase the yield of a hard butter produced from the resultant A without any reduction in the qualith thereof or to improve the quality of the hard butter without reducing the yield thereof. When the resultant B is reused as a part of the mixture A without hydrogenation, this effect cannot be achieved. Another effect of reuse of material C as a part of mixture A is that the amount of material B required to produce a desired amount of a hard butter can be reduced. Still another effect of reuse of material C as part of mixture A is that an inexpensive crude material can be used as material B and, even if such a crude material is used, the reactivity can be increased by reuse of material C. Furthermore, still another effect of reuse of material C as a part of mixture A is to reduce the reaction time and this effect can be enhanced by reuse of all or a low melting point fraction of the resultant A as a part of mixture A together with reuse of material C.

This reuse of the resultant A means that the transesterification reaction on material A is divided into multiple stages. In each stage, when a refreshed fatty acid source (i.e. material C) as well as a low melting point fraction of the resultant A are added to the mixture A, the transesterification can be quite efficiently carried out since the reaction time is considerably reduced and a loss such as glyceride having the desired composition is again subjected to the reaction to undergo decomposition and synthesis can be avoided. This reuse of all or a low melting point fraction of the resultant A as a part of the mixture A is very effective when the fatty acid residues at the $\alpha$-position of the material A are composed of at least 35 %, preferably at least 40 % of the fatty acid residues derived from an unsaturated fatty acid having 18 carbon atoms (oleic acid, linolic acid or linolenic acid). Thus, when the degree of unsaturation of material A is not less than the above, even if the transesterification reaction is completed in one reaction, it is difficult to obtain a hard butter of good quality unless a low melting point fraction is removed from the resultant A. Therefore, the above reuse of all or the low melting point fraction of the resultant A does not ado any extra step to the modification of fats and oils. On the contrary, the yield of a hard butter can be increased by such a reuse. In a conventional method, a low melting point fraction obtained by the fractionation of a glyceride can hardly be used as a material for the production of a hard butter since it contains a small amount of 2-unsaturated fatty acid residue-1,3-di-saturated fatty acid residue ingredient which is useful for a hard butter and a large amount of a material such as a diglyceride which reduces the quality of a hard butter. However, according to the present invention, a low melting point fraction of a glyceride can be reused and since it is a 2-unsaturated fatty acid residue it can be positively utilized in the production of a hard butter. Moreover, in the present invention, the content of diglyceride in a hard butter can be reduced by carrying out the transesterification reaction in as dry state as possible.

Before hydrogenation of the resultant B, if necessary, removal of a hydrogenation catalyst poison and refining of the resultant B may be effected. Hydrogenation can be carried out according to a standard method. The degree of hydrogenation if preferably such that at least 40 % of the unsaturated fatty acid residues at the position where the transesterification reaction occurs in the resultant B (the degree of unsaturation can be expressed by the iodine value (I.V.)) are saturated by hydrogenation and, most preferably, hydrogenation is carried out until a so-called fully hardened state is obtained (in general, I.V. < 1.). When the degree of hydrogenation is higher, more effective results in the above improvement of the quality and yield of a hard butter, a reduction in the amount of material B required for transesterification of material A and a reduction in the reaction time can be achieved.

In another preferred embodiment of the present invention, a fraction of a fatty acid having not more than 16 carbon atoms, or an ester thereof, is removed from the resultant B or material C before the reuse of material C. This operation can be carried out together with separation of the resultants A and B from the reaction mixture in single step (rectification). This embodiment is useful when the fatty acid residues at the position where the transesterification occurs in the mixture A are mainly composed of those derived from $C_{18}$ and $C_{16}$ fatty acids and a reduction of the content of the fatty acid residues derived from $C_{16}$ fatty acids is required in view of the fatty acid composition of the hard butter required to be obtained. With this embodiment, the effect of hydrogenation of the resultant B is enhanced. That is, when this embodiment is incorporated into the above basic method of the present invention, the improvement in the quality and yield of a hard butter, the reduction of the amount of material B required for transesterification of material A and for the production of a desired amount of a hard butter and the reduction of the reaction time are more efficient. Nowadays, since resources being rich in POP are more abundant and inexpensive than those being rich in SOS, in order to obtain a hard butter rich in POS, it is advantageous to use the resources rich in POP rather than those rich in SOS. Furthermore, in the production of high-purity OSO or POP which can be used as an improver of physical properties such as the adjustment of the melting point of cacao butter or a hard butter, it is advantageous to obtain SOS. Therefore, this embodiment is particularly effective when the fatty acid residues at the $\alpha$- position of the material A are composed of not less than 50 % of fatty acid residues derived from a saturated fatty acid having not more than 16 carbon atoms.

Still another preferred embodiment of the present invention is that the catalyst A is dipped in an ester before it is used. This can be effected, for example, by reuse of the catalyst B as a part or all of the catalyst A. That is, although the control of water content in the transesterification reaction system is very important as mentioned above, when a catalyst (i.e. enzyme) is freshly prepared, most of water in the reaction system is derived from the catalyst. On the other hand, the removal of water from an enzyme by heat-drying is limited due to the inactivation thereof and hence, usually, 1 to special % of water remains in the enzyme. We have previously found that when an enzyme is used repeatedly, the water content thereof decreases (European Patent Application No. 35883). This means that the water content of an enzyme can be decreased by dipping it in an oil and hence, the quality of a hard butter can be improved. Therefore, the reuse of the catalyst B is useful to reduce the water content of the transesterification reaction system. In this case, generally, only the water content of substrates (i.e. materials A and B) should be taken into consideration and a hard butter of good quality can be obtained when water content of the mixture A is maintained below 0.05 %.

In the method of the present invention, the ratio of material A to material B in the mixture A is generally 1 : 0.2 to 5. However, the total amount of the fatty acid source (i.e. material B) required for the transesterification reaction is very small due to the reuse of material C.

The following Examples further illustrate the present invention in detail but are not to be construed to limit the scope thereof. In the examples, all the "parts" and "%'s" are by weight unless otherwise stated.

Example 1

A commercially available lipase originated from Rhizopus japonicus (1.5 parts) was dissolved in cold water (5 parts) and diatomaceous earth (2.5 parts) was added to the solution with stirring. The resulting mixture was slowly dried at 20 °C under a reduced pressure to give an enzyme preparation containing 1.8 % of water.

A middle melting point fraction of palm oil (iodine value (I.V.)35, 11 % of unsaturated fatty acid residue and 77 % of palmitic acid residue at $\alpha$-position) (100 parts) and methyl stearate (purity 90 %, I.V. 0.5) (100 parts) were mixed and the mixture was dried under a reduced pressure to give a substrate containing 0.01 % of water.

The above-obtained enzyme preparation (10 parts) was added to the substrate (200 parts) and the mixture was stirred at 45 °C for 4 days with preventing water absorption. After removal of the enzyme preparation, the mixture was subjected to rectification to give a triglyceride fraction, a $C_{16}$ methyl ester fraction (purity of $C_{16}$ 90 %) and a $C_{18}$ methyl ester fraction (purity of $C_{18}$ 90%). The $C_{18}$ fraction was hydrogenated (hardened) untile I.V. became not more than 1 and thereto added fresh methyl stearate in such an amount that the resulting mixture became the same amount as that of methyl stearate initially used. The resulting mixture was again mixed with the fresh middle melting point fraction of palm oil in the ratio of 1 : 1 by weight to give another substrate. By using the resulting substrate and the fresh enzyme preparation, the same run was repeated. A high melting point portion was removed from the triglyceride fraction obtained in each run by solvent fractionation to give a hard butter. For comparison, the same run was repeated except that the $C_{18}$ fraction was not hydrogenated. I.V. of the methyl ester fraction recovered from each run is shown in Table 1.

Table 1

| Runs | Present invention I.V. | Comparative run I.V. |
|---|---|---|
| 1st | 3.8 | 3.9 |
| 2nd | 4.3 | 6.1 |
| 5th | 4.2 | 9.8 |

Every hard butter obtained in each run according to the present invention was similar to cacao butter and showed good results in a chocolate test. However, in the comparative runs, when the run was repeated 5 times in order to obtain a hard butter being similar to cacao butter, both high melting and low melting portions had to be removed from the resulting triglyceride fraction which resulted in lowering of yield since, after removal of only the high melting portion from the triglyceride fraction, the resultant had a low melting point (30.7° C, in case of present invention 33.5° C).

Besides, in order to transesterify the total amount of the middle melting point fraction of palm oil used in the 5 runs (500 parts) at once, 500 parts of methyl stearate was required in a conventional transesterification method. To the contrary, according to the present invention wherein $C_{18}$ methyl ester fraction was recovered after transesterification reaction, hydrogenated and reused, only about 260 parts of methyl stearate including operation loss was required.

The hard butter obtained in the 5th run of the present invention had the melting point of 33.5° C and I.V. of 35. The fatty acid composition thereof is shown in Table 2.

Table 2

| Fatty acid composition | $C_{14}$ | $C_{16}$ | $C_{18}$ | $C_{18:1}$ | $C_{18:2}$ |
|---|---|---|---|---|---|
| % | 0.5 | 28.1 | 33.9 | 34.9 | 2.6 |

Example 2

The same procedure as described in Example 1 was repeated except that an enzyme preparation was prepared by using a commercially available lipase originated from Rhizopus niveus and perlite; stearic acid was substituted for methyl stearate; the reaction was carried out at 69° C for 4 days; and separation of fatty acid fractions was effected by using a silica gel column. A hard butter being similar to cacao butter was constantly obtained in each run.

Example 3

A commercially available lipase originated from Rhizopus niveus (10 parts) was dissolved in cold water (40 parts) and Celite 545 (diatomaceous earch produced by Johns Marville Sales Corp., U.S.A.) (25 parts) was added to the solution with stirring. The resulting mixture was slowly dried at 15° C to give an enzyme preparation containing 2.5 % of water.

Refined shea butter (I.V. 58, 32 % of unsaturated fatty acid residue and 60 % of stearic acid residue at α-position) (25 parts) and stearic acid (containing 5.7 % of palmitic acid and 2.1 % of arachidic acid) ( 75 parts) were mixed and the mixture was dried at 105° C under a reduced pressure. Hexane (400 parts) was added to the dried mixture to give a substrate containing 0.01 % of water.

The above-obtained enzyme preparation (10 parts) was mixed with the substrate (200 parts) and the mixture was stirred at 45° C for 2 hours. The enzyme preparation was recovered from the reaction mixture. The recovered enzyme preparation was mixed with the fresh substrate (500 parts) and the transesterification reaction was carried out at 45° C with preventing water absorption.

After the reaction, hexane was distilled off from the reaction mixture and fatty acids were recovered from the reaction mixture by distillation. The fatty acids were hydrogenated until a fully hardened state was attained. The hydrogenated fatty acids were mixed with fresh refined shea fat ( 25 parts) and the transesterification reaction was repeated.

On the other hand, after removal of the fatty acids, the reaction mixture was subjected to fractionation to obtain a liquid-side fraction.

The transesterification reaction time and yield and melting point of the resulting liquid-side fraction are shown in Table 3.

Table 3

| Runs | Reaction time | Liquid-side fraction | |
|---|---|---|---|
| | | Yield | Melting point |
| 1st | 96 hours | 94 % | 38.5 °C |
| 2nd | 72 hours | 95 % | 38.9 °C |

When the liquid-side fraction was mixed with a middle melting point fraction of palm oil in the ratio of 1 : 1, a hard butter of good quality could be obtained.

Example 4

A commercially available lipase originated from Rhizopus japonicus (cell-bond enzyme) was dried under a reduced pressure until water content thereof became 1.5 %.

A middle melting point fraction of palm oil (I.V. 34) (35 parts), a liquid-side fractionated oil of shea fat (I.V. 67, 37 % of unsaturated fatty acid residue and 33 % of palmitic acid residue at $\alpha$-position) (65 parts) and fatty acid methyl ester (60 % of stearic acid and 40 % of palmitic acid) (200 parts) were mixed to give a substrate containing 0.01 % of water. The above enzyme (9 parts) was added to the substrate (300 parts) and the mixture was stirred at 45 °C for 7 days in a closed system. After recovery of the reaction mixture, a methyl ester fraction was separated by distillation and hydrogenated until I.V. thereof became less than 1. The hydrogenated ester fraction was again mixed with the triglyceride fraction recovered from the above reaction mixture and then the resulting mixture was again subjected to the transesterification reaction by using the enzyme recovered from the reaction mixture of the lst run. After reaction, the substrate was recovered and a methyl ester fraction was removed therefrom. Further, a high melting point frasction was removed from the resulting mixture by solvent fractionation to obtain a hard butter. The hard butter thus obtained was similar to cacao butter in respect of the fatty acid composition and the melting point thereof. The methyl esters removed from the substrate can be further reused in the transesterification after hydrogenation thereof.

Example 5

A commercially available pancreas lipase (5 g), polyvinyl alcohol cyanogenated with cyanogen bromide (2 g) and 0.1 M phosphate buffer (pH 7.5) (50 ml) were mixed and the mixture was allowed to stand in a refrigerator overnight. The mixture was filtered and dried under a reduced pressure to give a polyvinyl alcohol immobilized enzyme preparation (water content 1.7%).

Olive oil (81 % of unsaturated fatty acid residue at $\alpha$-position) (100 parts) and ethyl stearate (100 parts) were mixed and the mixture was dried under a reduced pressure to give a substrate (water content 0.015 %).

The above-obtained enzyme preparation (7.5 parts) was added to the substrate and the mixture was stirred at 45 °C for 3 days. The substrate was recovered and subjected to distillation to give an ethyl ester fraction and a triglyceride fraction. The ethyl ester fraction was hydrogenated (I.V. 0.5) and mixed with the triglyceride fraction. The mixture was again subjected to the transesterification reaction. After reaction, the substrate was recovered and treated as described above. This procedure was repeated 3 times and the resulting triglyceride fraction was separated into a solid-side fraction and a liquid-side fraction by solvent fractionation. The solid-side fraction had the melting point of 36.5 °C and I.V. of 36. The fatty acid composition thereof is shown in Table 4.

7

Table 4

| Fatty acid composition | $C_{16}$ | $C_{18}$ | $C_{18:1}$ | $C_{18:2}$ | $C_{20}$ |
|---|---|---|---|---|---|
| % | 10.5 | 49.2 | 38.1 | 1.2 | 0.3 |

When this fraction was mixed with a middle melting point fraction of palm oil in the ratio of 1 : 1, a cacao butter substitute having excellent properties could be obtained.

Example 6

A commercially available lipase originated from Rhizopus delemar (1 part) and diatomaceous earth (2 parts) were mixed and an appropriate amount of cold water was scattered thereon with stirring to form granules. The granules were dried at 15°C under a reduced pressure to give an enzyme preparation containing 1.5 % of water.

Refined safflower oil (high oleic) (89 % of unsaturated fatty acid residue at α-position) (100 parts) and methyl stearate (purity 93 %) (100 parts) were mixed and the mixture was dried under a reduced pressure to give a substrate (water content 0.01 %).

The above-obtained enzyme preparation (10 parts) was added to the substrate and the mixture was stirred at 45°C for 5 days in a closed system. The substrate was recovered and a methyl ester fraction was distilled off therefrom. The resulting triglyceride fraction was separated into a solid-side fraction and a liquid-side fraction by solvent fractionation.

The methyl ester fraction removed from the substrate was hydrogenated until a fully hardened state was attained and thereto added fresh methyl stearate in such an amount that the resulting ester mixture became 100 parts.

The ester mixture was added to a mixture of the above-obtained liquid side fraction (60 parts) and fresh refined safflower oil (40 parts) to give a substrate. The substrate was again subjected to the transesterification as described above. This procedure was repeated. I.V. of the methyl ester fraction recovered from each run as well as diglyceride content and yield of the high melting point portion of the triglyceride fraction obtained in each run are shown in Table 5.

Table 5

| Runs | Methyl ester fraction I.V. | Triglyceride fraction | |
|---|---|---|---|
| | | Diglyceride content (%) | Yield of high melting point portion (%) |
| 1st | 39.2 | 4.5 | 35 |
| 2nd | 30.1 | 5.2 | 40 |
| 3rd | 30.6 | 4.8 | 42 |
| 4th | 31.2 | 5.0 | 45 |
| 5th | 30.2 | 4.9 | 45 |

The high melting point portion of the triglyceride fraction obtained in 5th run could be used as a hard butter as such. In order to further improve quality thereof, an extremely high melting point portion and a liquid-side portion were removed therefrom to obtain a middle melting point portion. This portion had the melting point of 38.0°C and I.V. of 33 and showed excellent properties for a hard butter. The fatty acid composition is shown in Table 6.

Table 6

| Fatty acid composition | $C_{16}$ | $C_{18}$ | $C_{18:1}$ | $C_{18:2}$ | $C_{20}$ |
|---|---|---|---|---|---|
| % | 4.0 | 59.3 | 31.5 | 3.5 | 1.1 |

When this portion was mixed with an equal amount of a middle melting point fraction of palm oil, a hard butter of good quality useful in the chocolate production could be also obtained.

Example 7

When the same procedure as described in Example 6 was repeated except that water (0.3 %) was added to each run, diglyceride contents in the reaction mixtures of lst and 2nd runs were 10.3 % and 14.5 %, respectively.

## Claims

1. A method for the modification of a fat or oil wherein a mixture (mixture A) containing a glyceride-type fat or oil to be modified which contains not less than 3% of unsaturated fatty acid residues and not less than 50% of fatty acid residues having not more than 16 carbon atoms at the a-position as well as not less than 70% of oleic acid residues at the $\beta$-position thereof (material A), and a fatty acid or a nonglyceride-type ester thereof (material B) is selectively transesterified in the presence of a catalyst having a selective transesterification activity (catalyst A) in order to randomize or unify the distribution of fatty acid residues in the a-position of the reaction products; the resulting fat or oil (resultant A), fatty acid or nonglyceride-type ester thereof (resultant B) and catalyst (catalyst B) are recovered from the reaction mixture; a hard butter is produced from the resultant A; a part of all of the resultant B is hydrogenated, and prior to or after the hydrogenation, a fatty acid having not more than 16 carbon atoms or an ester thereof is removed therefrom, and the hydrogenerated product (material C) is reused as a part of the mixture A.

2. A method as claimed in claim 1 wherein all or a low melting point fraction of the (resultant A) is reused as a part of the mixture A.

3. A method as claimed in claim 1 or claim 2 wherein the water content of the transesterification reaction system is not more than 0.18% by weight based on the weight of mixture A.

4. A method as claimed in any one of claims 1 to 3 wherein catalyst A is previously treated by dipping it in a fatty ester.

5. A method as claimed in claim 4 wherein all or a part of catalyst B is reused as all or a part of catalyst A.

6. A method as claimed in any one of claims 1 to 5 wherein the water content of the mixture A is below 0.05% by weight.

Figure 1

% Saturated fatty
acid residue
$(C \geqq 18)$

% Saturated fatty
acid residue
$(C \leqq 16)$

% Unsaturated
fatty acid
residue